# EUROPEAN PATENT SPECIFICATION

(11) **EP 2 547 318 B1**
(45) Date of publication and mention of the grant of the patent: **05.09.2018**
(21) Application number: 11705873.5
(22) Date of filing: 24.02.2011
(51) Int. Cl.: A61K 8/81, A61Q 5/06, A61Q 5/02, A61Q 5/12

(54) **HAIR COMPOSITION COMPRISING A POLYMER DYE**
ZUSAMMENSETZUNG ZUR HAARBEHANDLUNG ENTHALTEND EINEN POLYMEREN FARBSTOFF
COMPOSITION DE TRAITEMENT CAPILLAIRE COMPRENANT UN COLORANT POLYMÈRE

(30) Priority: 10.08.2010 WO PCT/CN2010/075835; 19.03.2010 WO PCT/CN2010/071157
(43) Date of publication of application: 23.01.2013
(73) Proprietor: Unilever PLC, London EC4Y 0DY (GB); Unilever N.V., 3013 AL Rotterdam (NL)
(72) Inventor: BATCHELOR, Stephen, Norman, Merseyside CH63 3JW (GB); BIRD, Jayne, Michelle, Merseyside CH63 3JW (GB); CHEN, Wei, Shanghai 200333 (CN); DAS, Julie, Rosalyn, Merseyside CH63 3JW (GB); TAO, Qingsheng, Shanghai 200335 (CN); WANG, Jinfang, Shanghai 200335 (CN)
(74) Representative: James, Helen Sarah
(86) International application number: PCT/EP2011/052737
(87) International publication number: WO 2011/113677

(56) References cited:
- WO-A1-2009/090125
- WO-A2-2008/139312
- US-A- 3 915 635
- US-B1- 6 306 182

## Description

The present invention relates to a polymer dye and a method for its use in dying hair. WO2009/090125 discloses a polymeric hair dyes wherein the cationic charge is located in dye moiety.

WO2008/139312 discloses a polymer having a neutral dye Sudan III) bound to acrylamide, containing unreacted acrylamide units.

Despite the prior art there remains a need for improved hair dying compositions.

Accordingly, and in a first aspect, there is provided a hair care composition comprising a polymer dye obtainable by polymerizing:
(a) a first dye monomer which has a structure according to Formula I: 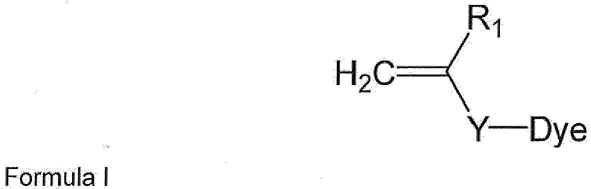 where Y is selected from -CONH- or -CO₂CH₂CH(OH)CH₂-NH-, and R₁ is selected from H, alkyl; aryl; benzyl; halogen; ester; acid amide; and, CN; and
   wherein the dye is negative or uncharged at pH8; and is selected from the class acid, basic, disperse or solvent dye bearing an -NH₂ group covalently bound to an aromatic group of the dye
(b) a second monomer according to a structure according to Formula II and Formula III: selected from 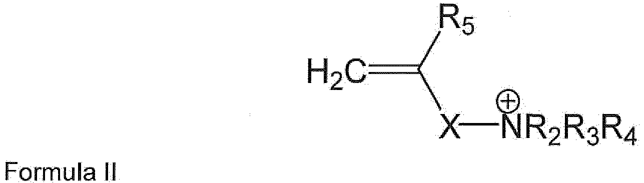 and 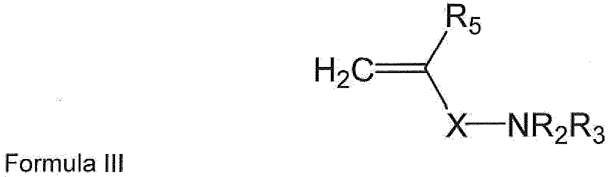 where X is -CO₂(CH₂)ₙ- where n = 1 to 6;
   where R₅ is selected from: H, alkyl; aryl; benzyl; halogen; ester; acid amide; and, CN, preferably R₅ is CH₃ or H; and
   where R₂, R₃, and R₄ are independently selected from H or alkyl, preferably at least two of R₂, R₃, and R₄ are alkyl.

Preferably, Y is -CO₂CH₂CH(OH)CH₂-NH-. Preferably, R₁ is selected from H or CH₃. Preferably, n=2. Preferred alkyl groups are C1 to C6 branched, linear or cyclic, most preferably CH₃ or C₂H₅.

Preferably, the dye is selected from the following chromophore classes: anthraquinone, azo, oxazine, azine, triphenodioxazine, triphenyl methane, xanthene and phthalocyanin, more preferably azo, anthraquinone and azine chromophore classes, most preferably azo and anthraquinone, most preferably, the dye is an anthraquinone.

The dye is negative or uncharged at pH 8. More preferably, the dye is uncharged at pH 8.

Monomer (a) is obtainable by reacting an alkene with a dye selected from the class acid, basic, disperse or solvent dye bearing an NH₂ group covalently bound to an aromatic group of the dye. Examples of such dyes are listed in the Color Index (Society of Dyers and Colourists and American Association of Textile Chemists and Colorists).

Preferably, the reactions are selected from: 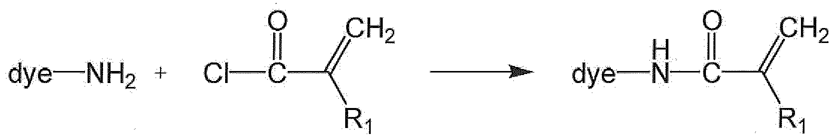 and

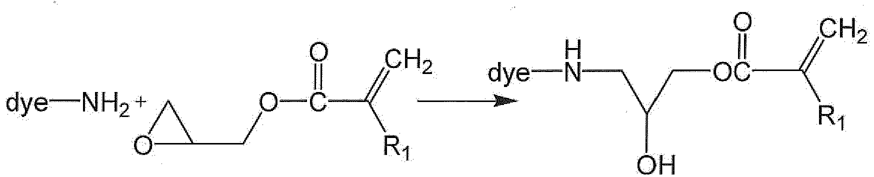

Preferred dyes containing -NH₂ groups for such reactions are selected from: acid violet 1; acid violet 3; acid violet 6; acid violet 11; acid violet 13; acid violet 14; acid violet 19; acid violet 20; acid violet 36; acid violet 36:1; acid violet 41; acid violet 42; acid violet 43; acid violet 50; acid violet 51; acid violet 63; acid violet 48; acid blue 25; acid blue 40; acid blue 40:1; acid blue 41; acid blue 45; acid blue 47; acid blue 49; acid blue 51; acid blue 53; acid blue 56; acid blue 61; acid blue 61:1; acid blue 62; acid blue 69; acid blue 78; acid blue 81:1; acid blue 92; acid blue 96; acid blue 108; acid blue 111; acid blue 215; acid blue 230; acid blue 277; acid blue 344; acid blue 117; acid blue 124; acid blue 129; acid blue 129:1; acid blue 138; acid blue 145; direct violet 99; direct violet 5; direct violet 72; direct violet 16; direct violet 78; direct violet 77; direct violet 83; food black 2; direct blue 33; direct blue 41; direct blue 22; direct blue 71; direct blue 72; direct blue 74; direct blue 75; direct blue 82; direct blue 96; direct blue 110; direct blue 111; direct blue 120; direct blue 120:1; direct blue 121; direct blue 122; direct blue 123; direct blue 124; direct blue 126; direct blue 127; direct blue 128; direct blue 129; direct blue 130; direct blue 132; direct blue 133; direct blue 135; direct blue 138; direct blue 140; direct blue 145; direct blue 148; direct blue 149; direct blue 159; direct blue 162; direct blue 163; food black 2; food black 1 wherein the acid amide group is replaced by NH₂; disperse blue 1; disperse blue 5; disperse blue 6; disperse blue 9; disperse blue 11; disperse blue 19; disperse blue 20; disperse blue 28; disperse blue 40; disperse blue 56; disperse blue 60; disperse blue 81; disperse blue 83; disperse blue 87; disperse blue 104; disperse blue 118; disperse violet 1; disperse violet 4, disperse violet 8, disperse violet 17, disperse violet 26; disperse violet 28; solvent violet 26; solvent blue 12; solvent blue 13; solvent blue 18; solvent blue 68; solvent blue 76; disperse red 4; disperse red 11; disperse red 15; disperse red 31; disperse red 53; disperse red 55; disperse red 60; disperse red 63; disperse red 86; disperse red 91; disperse red 92; disperse yellow 9; disperse yellow 11; acid red 33; acid red 30; acid red 34; acid yellow 7; acid yellow 9; acid yellow 66; acid green 20; acid green 33; acid brown 4; acid brown 9 and acid black 1.

Further preferred dyes are selected from mono-azo dyes which contain a phenyl group directly attached to the azo group, wherein the phenyl group has an NH₂ group covalent bound to it.

Preferably, monomer (a) comprises from 0.5 to 30 wt%, preferably from 5 to 20 wt% of the polymer.

Preferably, monomer (b) comprises from 5 to 99.5 wt% of the polymer. Most preferably (b) is dimethyl amino ethyl methacrylate.

Preferably, the composition of the invention comprises uncharged monomer units, preferably at from 5 to 99% wt% of the polymer. Preferably the monomer units are selected from acrylates and styrenes. Preferably they are present at 10-30wt%.

Preferably, the composition of the invention is a rinse-off composition. More preferably, the composition is a shampoo, conditioner or hair dye composition. Where the composition is a shampoo it preferably comprises from 5 to 50% cleansing surfactant. Preferably, the cleansing surfactant comprises anionic and amphoteric surfactant. Where the composition is a conditioning composition it preferably comprises a conditioning active such as fatty alcohols, fatty acids, fatty amides and fatty esters, silicones, cationic surfactants etc.

Preferably, the composition of the invention has a pH of from 3 to 9, preferably from 4 to 8.

Combination of polymer dyes may be used to obtain a wide colour palette with use of a limited number of dyes. Preferably a trichromate system consisting of a mixture of three polymer dyes. Preferably the trichromate system contains a combination of a blue or black polymer dye, a red polymer dye and a yellow polymer dye.

In a second aspect the present invention provides a method for dyeing hair by applying to the hair a composition according to any preceding claim.

### EXAMPLES

### Example 1 Synthesis of dye monomer

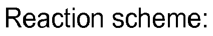

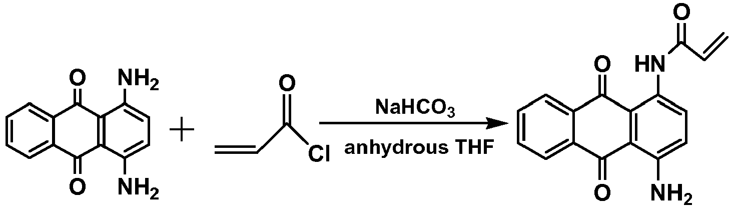

1,4-Diaminoanthraquinone (DAQ) was purchased from Aldrich, (90% technical grade) and used as supplied.

The dye monomer was prepared by the reaction of DAQ (1,4-Diaminoaquinone) and Ac (acryloyl chloride) in the presence of sodium dicarbonate. A mixture of 150ml anhydrous THF, 1g DAQ and 0.6g sodium dicarbonate was charged into 250ml three-necked round bottom flask equipped with a condenser, a dropping funnel, and a magnetic stirring bar. The flask was then maintained at R.T. while 0.38g Ac dissolved in 5ml anhydrous THF was added from the dropping funnel for 3h. The reaction mixture was stirred at 25°C for another 20h. The reaction mixture was filtered to remove the insoluble solids occurred during the reaction process 20h later, the clear solution was dried by using rotary evaporation pressure reduced meter, then violet powder was obtained and washed with water for three times, the powder was completely dried under vacuum at 60°C for 24h. The structure of DAQ-AC was confirmed using NMR and showed that the reaction had gone to greater than 88% completion. Consequently greater than 88% of the anthraquinones contained one NHCOCH=CH₂ groups.

### Example 2 Synthesis of dye containing polymers

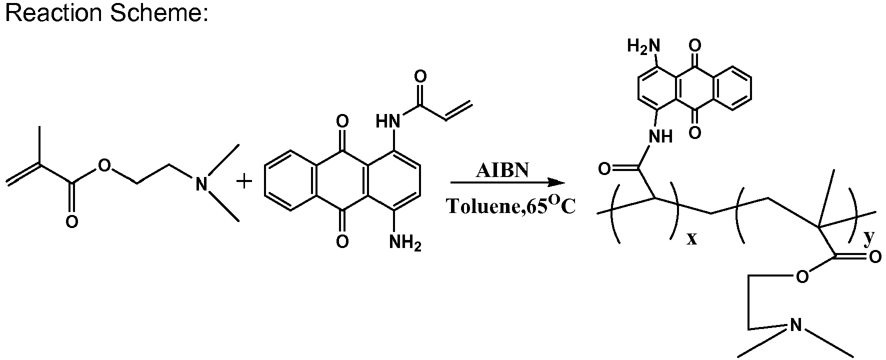

A solution of 3g dimethyl amino ethyl methacrylate (DMAEMA), 0.03g DAQ-Ac and 0.06g AIBN in 10ml toluene was charged into a dry N₂ gas purged two-necked tube with a condenser and a magnetic stirring bar. The tube was sealed and placed in a regulated thermostat bath at 65°C for 24hours. The solution was precipitated in five-fold excess of petroleum ether for three times after the reaction, followed by drying under vacuum at 40°C for 24h. The composition of the co-polymers was regulated via the control of the primary usages of dye monomer and functional monomer. In the experiments, the amount of dye monomer added were 10 wt % respectively.

### Example 3 Synthesis of dye containing polymers

Analagous reactions to example 1 and 2 were done using acid blue 14 and methylene violet RAX to produce the following dye polymers, the latter not being according to the invention.

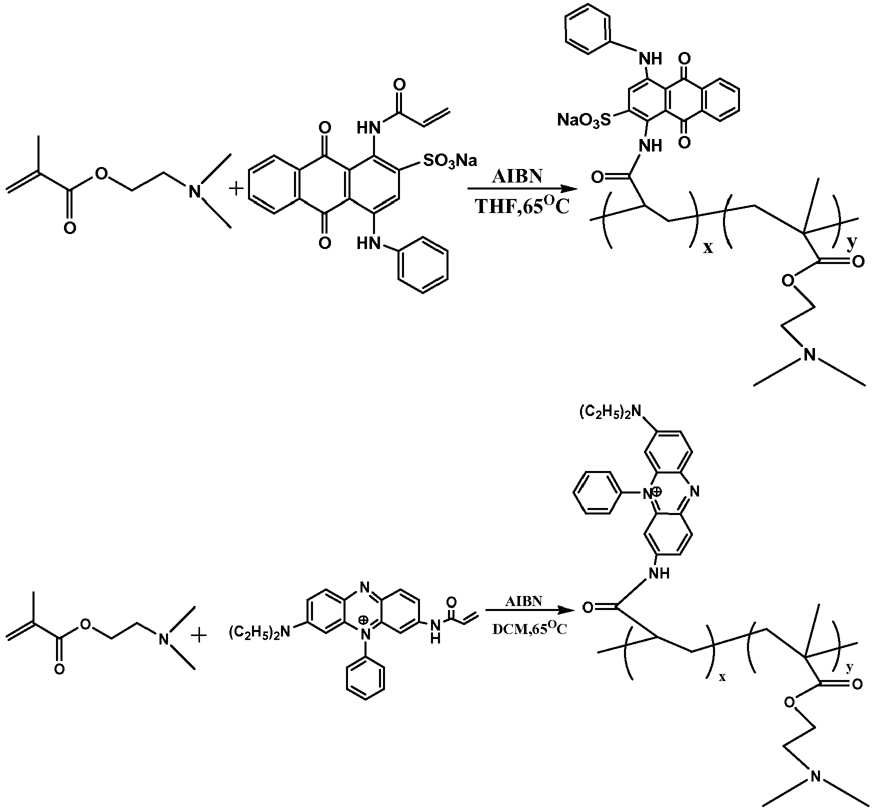

### Example 3 UV-VIS of dye polymers

The UV-Vis spectra of the dye polymers of example 2 were recorded in demineralised water at 1g/L dye polymer. The results are given in the table below. For C11 1g/L Linear alkyl benzene sulphonate surfactant was added.

| Code | Dye-monomer | λmax in range 400-700nm | Absorbance (1cm) @ λmax for 1g/L in water |
|---|---|---|---|
| C7 | 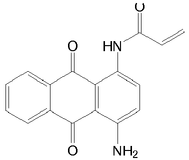 | 546 | 1.10 |
| C11 | 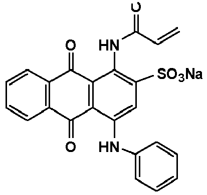 | 534 | 1.37 |
| C15 | 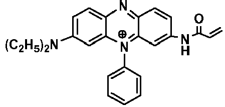 | 557 | 4.37* |

| | | | |
|---|---|---|---|
| * measured at 50% dilution | | | |

C15 is not according to the invention.

### Example 3 deposition to wool

Bleached knitted wool swatches were agitated in demineralised water for 30 minutes at 293K, to which 20ppm of C7, C11, C15 had been added and 40 ppm of C19 respectively. The weight ratio of wool to liquor was 1:30. The wool swatches were removed and dried in the air, then the colour measured using a reflectometer (UV excluded) and expressed as the CIE L*a*b* values. The results are given in the table below:-

| | L* | a* | b* |
|---|---|---|---|
| Control | 86.5 | 0.1 | 17.2 |
| C7 | 78.9 | 6.5 | 8.6 |
| C11 | 81.8 | 2.9 | 11.4 |
| C15 | 69.6 | 13.8 | 0.9 |

All the dye polymers dyed the wool as shown by changes in the L*, a* and b* to the control agitated in water alone.

### Example 4 deposition to human hair

Human hair swatches were agitated in demineralised water for 30 minutes at 293K, to which 20ppm of C7, C11, C15 had been added. The weight ratio of hair to liquor was 1:30. Grey-brown and blonde hair swatches were used. The hair swatches were removed and dried in the air, then the colour measured using a reflectometer (UV excluded) and expressed as the CIE L*a*b* values. The results are given in the table below and are average of 3 repeats.:-

| Grey-brown | L* | a* | b* |
|---|---|---|---|
| control | 39.4 | 0.9 | 6.4 |
| C7 | 40.9 | 1.7 | 5.5 |
| C11 | 39.0 | 1.6 | 5.5 |
| C15 | 36.8 | 4.0 | 2.9 |

| blonde | L* | a* | b* |
|---|---|---|---|
| Control | 86.5 | 0.1 | 17.2 |
| C7 | 78.9 | 6.5 | 8.6 |
| C11 | 81.8 | 2.9 | 11.4 |
| C15 | 69.6 | 13.8 | 0.9 |

### Example 5 dye polymer synthesis

The following dye-polymers were synthesised by polymerisation of the monomers as listed in the table below:

| | P1 | P2 | P3 | P4 | P5 | P6 |
|---|---|---|---|---|---|---|
| monomer | Weight% monomer used | | | | | |
| 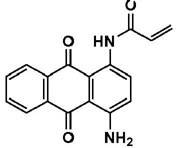 | 5 | | | 5 | 5 | |
| 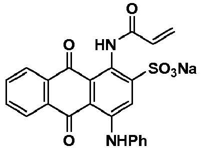 | | 5 | | | | |
| 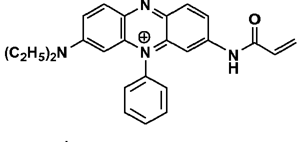 | | | 5 | | | |
| 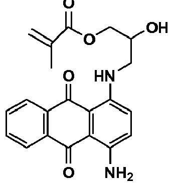 | | | | | | 5 |
| 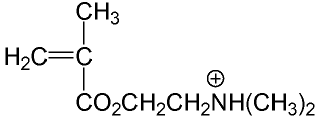 | 95 | 95 | 95 | | 75 | 95 |
| 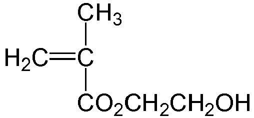 | | | | 75 | | |
| 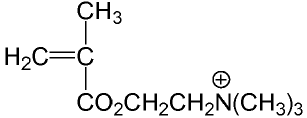 | | | | 20 | | |
| styrene | | | | | 20 | |

Polymer 3 is not according to the invention.

The dye monomers were synthesis by reacting the NH₂ group of the corresponding dye with acryloyl chloride or 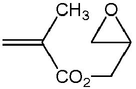 as appropriate.

### Example 6: Dyeing Performance

A 1 wt% solution of the dye polymers was made in demineralised water. A 3 by 10cm knitted woollen swatch (sheep) was place into the dye-polymer solution and agitated for 20 seconds, removed and dried. The colour of the wool was assessed by measuring the reflectance of the swatch using a reflectomer and expressing the colour as CIE L*a*b* values and as a ΔE value relative to an undyed control. The swatch was then rinsed in water for 30 seconds under a running tap, dried and the colour remeasured. The swatch was then washed in shampoo, rinsed, dried and re-measured. The results are shown below

| | ΔE | | | | | |
|---|---|---|---|---|---|---|
| | P1 | P2 | P3 | P4 | P5 | P6 |
| Initial | 38 | 41 | 51 | 23 | 24 | 23 |
| Water rinsed | 35 | 29 | 51 | 23 | 22 | 21 |
| Shampooed | 33 | - | 49 | 20 | 17 | 18 |

The Shampoo used was of the following formula: 20wt% Sodium lauryl ether sulphate; 5.3wt% cocoamidopropyl betaine; 6.5wt% Mirasheen A-220 (ex Rhodia) which is a mix of Glycol Distearate, Ammonium Laureth Sulfate, Amonium Lauryl Sulfate, Cocamide MEA, Ammonium Xylene Sulfonate; remainder minors and water.

### Example 7

The following are compositions made by standard processes.

**Shampoo**

| **Component** | **%ad** | **%** |
|---|---|---|
| Sodium Laureth Sulphate | 70 | 17.14 |
| Cocoamidopropyl Betaine | 30 | 5.33 |
| Carbomer | 100 | 0.4 |
| Glycol Distearate | 35 | 4.0 |
| Dimethiconol emulsion | 50 | 4.0 |
| Guar Hydroxypropyl Trimonium Chloride | 100 | 0.2 |
| Parfum | 100% | 0.8 |
| DMDM Hydantoin and 3-iodo-2propylnylbutyl carbamate | 50% | 0.2 |
| Sodium chloride | 100% | Visc. |
| Dye-polymer | 100% | 0.5 |
| Aqua | | q.s.to 100 |

**Conditioner**

| **Ingredient** | **Wt%** |
|---|---|
| Water | To 100 |
| Lactic acid | 0.38 |
| Methyl parahydroxybenzoate | 0.2 |
| Stearyl alcohol | 5 |
| Behenyltrimmonium chloride | 0.87 |
| LexamineS-13 (100%TAS) | 1.25 |
| Silicone emulsion | 2.5 |
| Perfume | 0.5 |
| Dye-polymer | 2 |

The dye-polymer was a 1:1:1 mixture of 3 dye polymers, all based on 95wt% dimethyl amino ethyl methacrylate, with 5wt% dye monomer (a).
Polymer A: dye monomer made by reaction of acroyl chloride with disperse red 55
Polymer B: dye monomer made by reaction of acroyl chloride with disperse yellow 11.
Polymer C: dye monomer made by reaction of acroyl chloride with disperse blue 56.

Analagous formulation were created but with dye monomer made by reaction of the disperse dye with

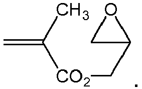

## Claims

1. Hair composition comprising a polymer dye, said polymer dye being synthesized by polymerizing:
(a) a first dye monomer which has a structure according to Formula I: 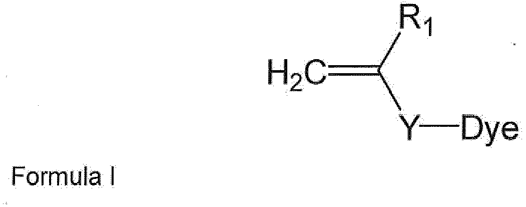 where Y is selected from -CONH- or -CO₂CH₂CH(OH)CH₂-NH- and R₁ is selected from H, alkyl; aryl; benzyl; halogen; ester; acid amide; and, CN, and wherein the dye is negative or uncharged at pH8; and is selected from the class acid, basic disperse or solvent dye bearing an -NH₂ group covalently bound to an aromatic group of the dye; and
(b) a second monomer according to a structure according to Formula II: and Formula III: selected from 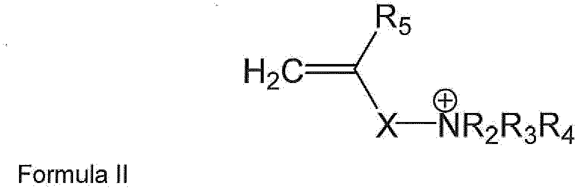 and 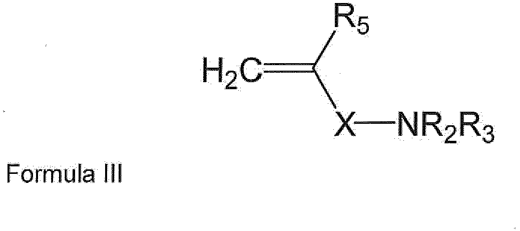
where X is -CO₂(CH₂)ₙ- where n = 1 to 6;
where R₅ is selected from: H, alkyl; aryl; benzyl; halogen; ester; acid amide; and, CN, preferably R₅ is CH₃ or H; and
where R₂, R₃, and R₄ are independently selected from H or alkyl, preferably at least two of R₂, R₃, and R₄ are alkyl.

2. Composition according to claim 1 wherein the dye is selected from the following chromophore classes: anthraquinone, azo, oxazine, azine, triphenodioxazine, triphenyl methane, xanthene and phthalocyanin, more preferably azo, anthraquinone and azine chromophore classes, most preferably azo and anthraquinone, most preferably, the dye is an anthraquinone.

3. Composition according to any preceding claim wherein the dye is uncharged at pH8.

4. Composition according to any preceding claim wherein monomer (a) comprises from 0.5 to 30 wt%, preferably from 5 to 20 wt% of the polymer.

5. Composition according to any preceding claim wherein monomer (b) comprises from 5 to 99.5 wt% of the polymer.

6. Composition according to any preceding claim which is a rinse-off composition.

7. Composition according to any preceding claim having a pH of from 3 to 9, preferably from 4 to 8.

8. Method for dyeing hair by applying to the hair a composition according to any preceding claim.

## Patentansprüche

1. Zusammensetzung zur Haarbehandlung, umfassend einen Polymerfarbstoff, wobei der Polymerfarbstoff synthetisiert wird durch Polymerisieren
(a) eines ersten Farbstoffmonomers, das eine Struktur gemäß der Formel I 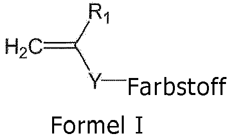 aufweist,
wo Y aus -CONH- oder -CO₂CH₂CH(OH)CH₂-NH- ausgewählt ist und
R₁ aus Wasserstoff, Alkyl, Aryl, Benzyl, Halogen, Ester, Säureamid und CN ausgewählt ist und
wobei der Farbstoff bei pH 8 negativ oder ungeladen ist und aus der Klasse saurer, basischer, Dispersions- oder Lösungsmittelfarbstoff ausgewählt ist, der eine kovalent an eine aromatische Gruppe des Farbstoffs gebundene -NH₂-Gruppe trägt, und
(b) eines zweiten Monomers gemäß einer Struktur gemäß der Formel II und III, ausgewählt aus 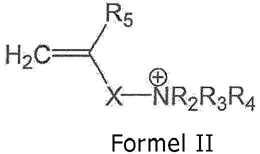 und 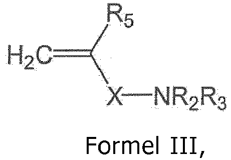
wo X -CO₂(CH₂)ₙ- ist, wo n = 1 bis 6 ist,
wo R₅ ausgewählt ist aus: H, Alkyl, Aryl, Benzyl, Halogen, Ester, Säureamid und CN, wo R₅ vorzugsweise CH₃ oder H ist, und
wo R₂, R₃ und R₄ unabhängig voneinander aus H oder Alkyl ausgewählt sind, vorzugsweise mindestens zwei von R₂, R₃ und R₄ Alkyl sind.

2. Zusammensetzung nach Anspruch 1, wobei der Farbstoff aus den folgenden Chromophor-Klassen ausgewählt ist: Anthrachinon, Azo, Oxazin, Azin, Triphenodioxazin, Triphenylmethan, Xanthen und Phthalocyanin, bevorzugter Azo-, Anthrachinon- und Azin-Chromophor-Klassen, höchst bevorzugt Azo und Anthrachinon, wobei der Farbstoff höchst bevorzugt ein Anthrachinon ist.

3. Zusammensetzung nach irgendeinem vorhergehenden Anspruch, wobei der Farbstoff bei pH 8 ungeladen ist.

4. Zusammensetzung nach einem vorhergehenden Anspruch, wobei das Monomer (a) von 0,5 bis 30 Gew.-%, vorzugsweise von 5 bis 20 Gew.-% des Polymers umfasst.

5. Zusammensetzung nach irgendeinem vorhergehenden Anspruch, wobei Monomer (b) von 5 bis 99,5 Gew.-% des Polymers umfasst.

6. Zusammensetzung nach irgendeinem vorhergehenden Anspruch, welche eine abspülbare Zusammensetzung darstellt.

7. Zusammensetzung nach irgendeinem vorhergehenden Anspruch mit einem pH von 3 bis 9, vorzugsweise von 4 bis 8.

8. Verfahren zum Färben von Haar durch Auftragen einer Zusammensetzung nach einem vorhergehenden Anspruch auf das Haar.

## Revendications

1. Composition pour les cheveux comprenant un colorant polymère, ledit colorant polymère étant synthétisé par polymérisation :
(a) d'un premier monomère de colorant qui présente une structure selon la Formule I : 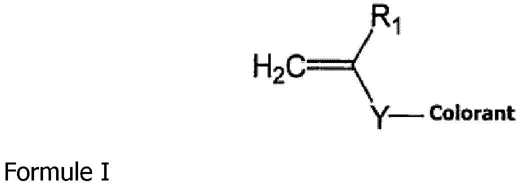 où Y est choisi parmi -CONH- ou -CO₂CH₂CH(OH)CH₂-NH- et R₁ est choisi parmi H, un groupe alkyle ; aryle ; benzyle ; un halogène ; un ester ; un amide d'acide ; et, CN, et dans laquelle le colorant est négatif ou non chargé à pH8 ; et est choisi parmi un colorant de classe acide, dispersé basique ou solvant portant un groupe -NH₂ lié de manière covalente à un groupe aromatique du colorant ; et
(b) un second monomère selon une structure selon la Formule II et la Formule III : choisi parmi 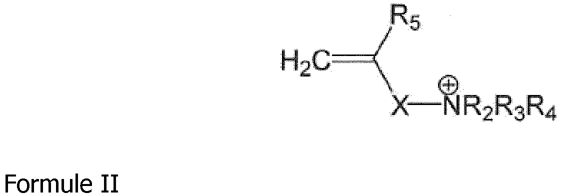 et 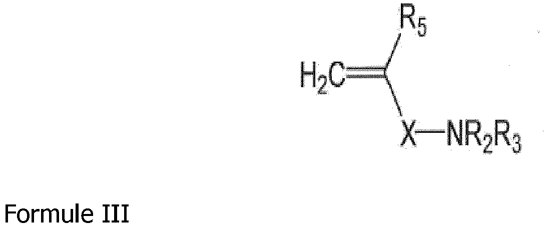
où X est -CO₂(CH₂)ₙ- où n = 1 à 6 ;
où R₅ est choisi parmi : H, un groupe alkyle ; aryle ; benzyle ; un halogène ; un ester ; un amide d'acide ; et, CN, de préférence R₅ est CH₃ ou H ; et
où R₂, R₃, et R₄ sont indépendamment choisis parmi H ou un groupe alkyle, de préférence au moins deux de R₂, R₃, et R₄ sont un groupe alkyle.

2. Composition selon la revendication 1, dans laquelle le colorant est choisi parmi les classes de chromophore suivantes : anthraquinone, azo, oxazine, azine, triphénodioxazine, triphénylméthane, xanthène et phthalocyanine, de préférence les classes de chromophores azo, anthraquinone et azine, encore mieux azo et anthraquinone, bien mieux encore, le colorant est une anthraquinone.

3. Composition selon l'une quelconque des revendications précédentes, dans laquelle le colorant est non chargé à pH8.

4. Composition selon l'une quelconque des revendications précédentes, dans laquelle le monomère (a) représente de 0,5 à 30 % en masse, de préférence de 5 à 20 % en masse du polymère.

5. Composition selon l'une quelconque des revendications précédentes, dans laquelle le monomère (b) représente de 5 à 99,5 % en masse du polymère.

6. Composition selon l'une quelconque des revendications précédentes qui est une composition sans rinçage ;

7. Composition selon l'une quelconque des revendications précédentes ayant un pH de 3 à 9, de préférence de 4 à 8.

8. Procédé de coloration de cheveux par application aux cheveux d'une composition selon l'une quelconque des revendications précédentes.
